# EUROPÄISCHE PATENTSCHRIFT

(11) **EP 3 270 760 B1**
(45) Veröffentlichungstag und Bekanntmachung des Hinweises auf die Patenterteilung: **21.11.2018**
(21) Anmeldenummer: 16708635.4
(22) Anmeldetag: 03.03.2016
(51) Int. Cl.: A61B 1/00

(54) **ELEKTRISCHES VERBINDUNGSSTÜCK FÜR EIN ENDOSKOP UND VERFAHREN ZUM HERSTELLEN EINER ELEKTRISCHEN VERBINDUNG IN EINEM ENDOSKOP**
ELECTRICAL CONNECTION PIECE FOR AN ENDOSCOPE AND METHOD FOR ESTABLISHING AN ELECTRICAL CONNECTION IN AN ENDOSCOPE
PIÈCE ÉLECTRIQUE DE LIAISON POUR UN ENDOSCOPE ET PROCÉDÉ DE PRODUCTION D'UNE LIAISON ÉLECTRIQUE DANS UN ENDOSCOPE

(30) Priorität: 18.03.2015 DE 102015204884
(43) Veröffentlichungstag der Anmeldung: 24.01.2018
(73) Patentinhaber: Olympus Winter & Ibe GmbH, 22045 Hamburg (DE)
(72) Erfinder: WIETERS, Martin, 22885 Barsbüttel (DE); SCHNITGER, Jens, 22587 Hamburg (DE)
(74) Vertreter: Seemann & Partner Patentanwälte mbB
(86) Internationale Anmeldenummer: PCT/EP2016/054503
(87) Internationale Veröffentlichungsnummer: WO 2016/146393

(56) Entgegenhaltungen:
- EP-A1- 2 677 736
- WO-A1-2014/065099
- DE-A1-102012 202 133
- US-A1- 2011 249 106

## Beschreibung

Die Erfindung betrifft ein elektrisches Verbindungsstück für ein Endoskop, umfassend eine wenigstens teilweise flexible Leiterplatte mit einem zentralen Abschnitt, der von Kontaktöffnungen für Kontaktstifte einer hermetisch abgeschlossenen Durchführung einer bildgebenden Einheit des Endoskop durchsetzt ist, wobei die Leiterplatte ferner einen biegbaren ersten Arm und einen biegbaren zweiten Arm umfasst, die in verschiedenen Richtungen von dem zentralen Abschnitt abzweigen, wobei der erste Arm an seinem dem zentralen Abschnitt abgewandten Ende eine erste flache Endfläche umfasst und der zweite Arm an seinem dem zentralen Abschnitt abgewandten Ende eine zweite flache Endfläche umfasst, wobei Leiterbahnen zwischen den Kontaktöffnungen und in den Endflächen vorhandenen ersten elektrischen Kontaktierungsflächen verlaufen. Ferner betrifft die Erfindung ein Endoskop mit einem Schaft und einer in dem Schaft angeordneten hermetisch abgeschlossenen bildgebenden Einheit. Die Erfindung betrifft ebenso ein Verfahren zum Herstellen einer elektrischen Verbindung in einem Endoskop.

Bei Endoskopen, beispielsweise Videoendoskopen befindet sich an der distalen Spitze eines Endoskopschafts eine Optik, beispielsweise ein geradeaus blickendes oder seitwärts blickendes Objektiv. An dieses schließt sich ein Bildsensor an, der das empfangene Licht in elektrische Signale umwandelt, die über geeignete elektrische Leitungen nach proximal weitergeleitet werden. Die elektrischen Leitungen, mit denen die Signale im Inneren des Endoskopschafts übertragen werden, können Kabel mit mehreren geschirmten oder ungeschirmten Litzen, flexible Leiterplatten oder dgl. sein.

Bei einigen Endoskopen befinden sich sowohl die Optik als auch der Bildsensor in einem hermetisch abgedichteten Raum. Ein solches Endoskop ist beispielsweise aus DE 10 2012 202 133 A1 bekannt. Allgemein wird eine optische Baugruppe an der distalen Spitze des Endoskops auch als "R-Unit" bezeichnet. Sie beinhaltet die optischen Linsensysteme und gegebenenfalls einen optischen Flächensensor, beispielsweise einen CCD- oder einen CMOS-Sensor. Zur Kontaktierung einer solchen R-Unit ist eine hermetisch dichte Durchkontaktierung der elektrischen Leitungen vorhanden. Eine solche hermetisch dichte elektrische Durchführung wird beispielsweise realisiert, indem Metallpins bzw. Kontaktstifte in einen Glasträger eingegossen werden. Die zur Signalübertragung verwendeten elektrischen Leitungen werden beispielsweise direkt an diese Metallpins angelötet.

Es ist eine Aufgabe der Erfindung, ein elektrisches Verbindungsstück für ein Endoskop, ein Endoskop sowie ein Verfahren zum Herstellen einer elektrischen Verbindung in einem Endoskop anzugeben, welches flexibel im Hinblick auf die Kontaktierung ist.

Die Aufgabe wird gelöst durch ein elektrisches Verbindungsstück für ein Endoskop, umfassend eine wenigstens teilweise flexible Leiterplatte mit einem zentralen Abschnitt, der von Kontaktöffnungen für Kontaktstifte einer hermetisch abgeschlossenen Durchführung einer bildgebenden Einheit des Endoskops durchsetzt ist, wobei die Leiterplatte ferner einen biegbaren ersten Arm und einen biegbaren zweiten Arm umfasst, die in verschiedenen Richtungen von dem zentralen Abschnitt abzweigen, wobei der erste Arm an seinem dem zentralen Abschnitt abgewandten Ende eine erste flache Endfläche umfasst und der zweite Arm an seinem dem zentralen Abschnitt abgewandten Ende eine zweite flache Endfläche umfasst, wobei Leiterbahnen zwischen den Kontaktöffnungen und in den Endflächen vorhandenen ersten elektrischen Kontaktierungsflächen verlaufen, wobei das elektrische Verbindungsstück dadurch fortgebildet ist, dass für die Kontaktöffnungen eine bezüglich einer Symmetrieachse spiegelsymmetrische Kontaktbelegung vorgesehen ist und ferner der erste Arm eine der ersten Endfläche gegenüberliegende flache dritte Endfläche umfasst und der zweite Arm eine der zweiten Endfläche gegenüberliegende flache vierte Endfläche umfasst, wobei weitere Leiterbahnen zwischen den Kontaktöffnungen und den in der dritten und der vierten Endfläche vorhandenen zweiten elektrischen Kontaktierungsflächen verlaufen und wobei die ersten elektrischen Kontaktierungsflächen zur Kontaktierung mit einer ersten Kontaktierungstechnik und/oder Kontaktbelegung und die zweiten elektrischen Kontaktierungsflächen zur Kontaktierung mit einer davon verschiedenen zweiten Kontaktierungstechnik und/oder Kontaktbelegung ausgestaltet sind.

Im Kontext der vorliegenden Beschreibung werden unter einander gegenüberliegenden Endflächen solche Flächen verstanden, welche auf einander gegenüberliegenden Flachseiten des jeweiligen Arms angeordnet sind. Insbesondere sind die einander gegenüberliegenden Endflächen in einer Richtung senkrecht zu ihrer Oberfläche zueinander zumindest näherungsweise deckungsgleich ausgestaltet bzw. angeordnet. Das Endoskop ist insbesondere ein Videoendoskop. Die bildgebende Einheit ist insbesondere eine Videoeinheit oder eine R-Unit. Das elektrische Verbindungsstück ist also insbesondere für ein Videoendoskop mit einer hermetisch abgeschlossenen Videoeinheit, welche sich in einem Schaft des Videoendoskops befindet, eingerichtet bzw. verwendbar.

Vorteilhaft erlaubt das elektrische Verbindungsstück eine flexible Kontaktierung und/oder Kontaktbelegung der Kontaktierungsflächen, was bedeutet, dass die hierzu verwendete Kontaktierungstechnik und/oder Kontaktbelegung im Voraus nicht festgelegt werden muss. Erst zu dem Zeitpunkt, zu dem das elektrische Verbindungsstück mit der bildgebenden Einheit des Endoskops verbunden wird, ist eine entsprechende Auswahl zu treffen. Das elektrische Verbindungsstück ist also für unterschiedliche Anschlussarten geeignet, wobei ein einziges Leiterplattendesign bzw. ein einziges elektrisches Verbindungsstück in einer Vielzahl verschiedener Endoskoptypen zum Einsatz kommt. Beispielsweise kann wahlweise ein hochauflösendes Videoendoskop mit einem 2- oder 3-CCD-System oder auch ein 3D-Videoendoskop kontaktiert werden. Dies senkt die Designkosten und die Teilepreise auch bei kleinen Stückzahlen. Durch die ermöglichte Gleichteilverwendung, welche durch die alternative Anwendbarkeit unterschiedlicher Kontaktierungstechniken oder Kontaktbelegungen bei ein und demselben elektrischen Verbindungsstück möglich ist, werden ferner bei größeren Stückzahlen die Ersatzteil- und Lagerkosten gesenkt.

Es ist vorteilhaft möglich, beispielsweise wenn die Kontaktierungsflächen mit Litzen eines Kabels verlötet werden, das Verlöten vorzunehmen, wenn die beiden Arme des elektrischen Verbindungsstücks noch nicht aufeinandergelegt sind. Dies betrifft auch eine Verbindung zwischen den Kontaktstiften der bildgebenden Einheit und den Durchführungen im zentralen Abschnitt der Leiterplatte. Die Durchführungen und die Kontaktflächen liegen offen zutage, so dass ein Verlöten ohne die ggf. hinderlich angeordneten Arme vornehmbar ist. Auch kann vorteilhaft eine automatische Verlötung erfolgen.

Gemäß einer vorteilhaften Ausführungsform ist das elektrische Verbindungsstück dadurch fortgebildet, dass die Arme in entgegengesetzten Richtungen von dem zentralen Abschnitt abzweigen und die Symmetrieachse zumindest näherungsweise parallel oder senkrecht zu einer gemeinsamen Längserstreckungsrichtung der Arme verläuft. Je nach Anordnung der Symmetrieachse wird das elektrische Verbindungsstück nach Wahl der gewünschten Kontaktierungstechnik um seine Längsachse gedreht oder quer dazu gewendet. Es ist ebenso möglich, eine Kontaktbelegung vorzusehen, die sowohl parallel als auch senkrecht zu der Längserstreckungsrichtung symmetrisch ist. Somit kann das elektrische Verbindungsstück beliebig gewendet oder gedreht werden, ohne dass eine falsche Kontaktierung möglich ist. Die Handhabung ist somit besonders einfach und die Gefahr einer fehlerhaften Montage wird auf ein Minimum begrenzt.

Ferner ist das elektrische Verbindungsstück insbesondere dadurch fortgebildet, dass wahlweise die ersten Kontaktierungsflächen in der ersten und zweiten Endfläche oder die zweiten Kontaktierungsflächen in der dritten und vierten Endfläche mit Kontakten eines weiteren elektrischen Verbindungselements, insbesondere eines oder mehrerer Kabel, einer oder mehrerer flexibler Leiterplatten oder eines oder mehrerer Stecker, verbindbar oder verbunden sind.

Der zentrale Abschnitt der Leiterplatte weist eine erste und eine gegenüberliegende zweite Seite auf. Die erste Seite des zentralen Abschnitts ist mit den ersten elektrischen Kontaktierungsflächen auf einer gemeinsamen Flachseite der Leiterplatte angeordnet. Entsprechend ist die zweite Seite des zentralen Abschnitts mit den zweiten elektrischen Kontaktierungsflächen auf einer gemeinsamen Flachseite der Leiterplatte angeordnet. Abhängig von der gewünschten Kontaktierungstechnik, mit der das weitere elektrische Verbindungselement kontaktiert werden soll, wird die erste oder die zweite Flachseite des zentralen Abschnitts der Durchführung der bildgebenden Einheit zugewandt. Entsprechend werden der erste und der zweite Arm derart gebogen, dass die Arme zumindest mittelbar aufeinander liegen, so dass diese sich in einem der gegenüberliegenden Seite des zentralen Abschnitts zugewandten Halbraum befinden. Vor oder nach dem Biegevorgang findet eine Kontaktierung der Kontaktflächen mit der gewünschten Kontaktierungstechnik statt.

Ferner ist insbesondere vorgesehen, dass die erste und die davon verschiedene zweite Kontaktierungstechnik aus der folgenden Liste ausgewählt sind: Verlöten, Steckverbinden, Verbinden über Kantenvias. Selbstverständlich sind weitere allgemein bekannte Kontaktierungstechniken möglich.

Gemäß einer weiteren vorteilhaften Ausführungsform ist vorgesehen, dass der zentrale Abschnitt Grundfläche und/oder die Endflächen und die weiteren Endflächen der Leiterplatte versteift und/oder weniger flexibel als die Arme ist oder sind. Wenn der zentrale Abschnitt der Leiterplatte versteift oder weniger flexibel als die Arme ausgeführt ist, ist es möglich, diesen einfach auf die Kontaktstifte der hermetischen Durchführung aufzuschieben und dort zu fixieren. Dies erleichtert auch das anschließende Verlöten der Kontaktstifte. Eine versteifte oder weniger flexible Ausführung der Endflächen der Arme erleichtert das Verbinden mit dem weiteren elektrischen Verbindungselement.

Das elektrische Verbindungsstück ist ferner insbesondere dadurch weitergebildet, dass der erste Arm und der zweite Arm zumindest näherungsweise eine gleiche Länge aufweisen und zumindest abschnittsweise flächig aufeinander legbar oder gelegt sind, wobei die erste und die zweite Endfläche oder die dritte und die vierte Endfläche im aufeinandergelegten Zustand von dem zentralen Abschnitt weg weisen und im aufeinandergelegten Zustand zusammen mit dem zentralen Abschnitt ein im Wesentlichen symmetrisches Dreieck bilden, wobei die aufeinander gelegten Endflächen im Wesentlichen auf einer zentralen Achse oder Drehachse der bildgebenden Einheit legbar sind oder liegen.

Als Länge der Arme wird eine Distanz zwischen dem jeweiligen Freiende des Arms und einem diesem zugewandten Rand des zentralen Abschnitts angesehen, an dem der zentrale Abschnitt in den betreffenden Arm übergeht.

Die oben genannte Anordnung der Arme relativ zu dem zentralen Abschnitt und insbesondere zu einer zentralen Achse oder Drehachse der bildgebenden Einheit erleichtert es, eine Bilddrehung der bildgebenden Einheit, beispielsweise einer Videoeinheit, bei gleichzeitig sicherer elektrischer Kontaktierung zu gewährleisten. Eine derartige achssymmetrische Anordnung bevorzugt keine der beiden möglichen Tordierungen, beispielsweise im oder entgegen dem Uhrzeigersinn, so dass sich ein gleichmäßiger Verschleiß beispielsweise einer sich anschließenden flexiblen Leiterplatte einstellt und keine der beiden möglichen Drehrichtungen bevorzugt wird.

Gemäß einer vorteilhaften Ausführungsform ist ferner vorgesehen, dass ein, insbesondere flächiger, Stabilisierungskörper umfasst ist, der zwischen den aufeinandergelegten Endflächen angeordnet ist, wobei der Stabilisierungskörper insbesondere ein elektrisch isolierendes Material umfasst und ferner insbesondere ein Material umfasst, mittels dessen ein Übersprechen von Signalen in den Leiterbahnabschnitten bzw. weiteren Leiterbahnabschnitten in den Endflächen des ersten Arms und des zweiten Arms verringert wird.

Insbesondere ist der Stabilisierungskörper im umgebogenen Zustand der Arme zwischen den jeweils aufeinanderliegenden Endflächen angeordnet. Der Stabilisierungskörper ist ferner insbesondere aus einem isolierenden Material hergestellt. Ferner insbesondere ist dieser aus einem Material hergestellt, welches das Übersprechen von Signalen verringert.

Der Stabilisierungskörper sorgt dafür, dass ein sicherer Kontakt mit dem weiteren elektrischen Verbindungsstück herstellbar ist. Gleichzeitig isoliert der Stabilisierungskörper die innen liegenden nicht genutzten Kontaktflächen voneinander.

Gemäß einer weiteren Ausführungsform ist vorgesehen, dass ein Verbindungselement umfasst ist, das die Endflächen durchdringt und miteinander verbindet, insbesondere als beidseitige pilzköpfige Vorsprünge am Stabilisierungskörper ausgebildet ist. Das Verbindungselement stellt eine einfache, schnell zu montierende und effiziente Haltfunktion für die beiden Arme aneinander dar. Das Verbindungselement kann ferner eine Verschraubung oder eine Vernietung sein. Es ist ebenso möglich, dass der Stabilisierungskörper elektrisch isolierend ausgeführt ist. Alternativ ist ebenfalls vorgesehen, dass die Arme an den innenliegenden Endflächen miteinander oder mit dem Stabilisierungskörper verklebt werden.

Gemäß einer weiteren Ausführungsform ist das elektrische Verbindungsstück dadurch fortgebildet, dass die Kontaktöffnungen des zentralen Abschnitts der Leiterplatte über Kontaktstifte der hermetischen Durchführung steckbar oder gesteckt und mit den Kontaktstiften verlötbar oder verlötet sind.

Insbesondere liegt der zentrale Abschnitt der Leiterplatte nicht flächig auf einer Oberfläche der hermetischen Durchführung auf. So wird verhindert, dass durch einen Kapillareffekt Lot in den vorhandenen Zwischenraum eindringt und ggf. zu Kurzschlüssen zwischen den Kontaktstiften führt.

Die Aufgabe wird ferner gelöst durch ein Endoskop, insbesondere ein Videoendoskop, mit einem Schaft und einer in dem Schaft angeordneten hermetisch abgeschlossenen bildgebenden Einheit, insbesondere einer Videoeinheit, wobei das Endoskop ferner ein elektrisches Verbindungsstück nach einem oder mehreren der genannten Ausführungsformen umfasst.

Auf das Endoskop treffen gleiche oder ähnliche Vorteile zu, wie sie bereits im Hinblick auf das elektrische Verbindungsstück erwähnt wurden. Auf eine erneute Vorstellung wird daher verzichtet.

Die Aufgabe wird ebenso gelöst durch ein Verfahren zum Herstellen einer elektrischen Verbindung in einem Endoskop, wobei ein elektrisches Verbindungsstück nach einem oder mehreren der genannten Ausführungsformen bereitgestellt und die erste oder die zweite Kontaktierungstechnik und/oder Kontaktbelegung zum Kontaktieren des elektrischen Verbindungsstücks ausgewählt wird, wobei
wenn die erste Kontaktierungstechnik und/oder Kontaktbelegung ausgewählt wird:
- eine erste Seite des zentralen Abschnitts, der mit den ersten elektrischen Kontaktierungsflächen auf einer gemeinsamen Flachseite der Leiterplatte angeordnet ist, der Durchführung der bildgebenden Einheit zugewandt und mit den Kontaktöffnungen auf deren Kontaktstifte gesteckt und verlötet wird,
- der erste Arm und der zweite Arm aufeinander zu gebogen und die dritte und vierte Endfläche der Arme aufeinandergelegt und miteinander verbunden werden, so dass die ersten elektrischen Kontaktierungsflächen nach außen zeigen,
- anschließend elektrisch leitende Verbindungen der ersten elektrischen Kontaktierungsflächen mit elektrischen Leitern eines weiteren elektrischen Verbindungselements gemäß der ersten Kontaktierungstechnik und/oder Kontaktbelegung hergestellt werden,
wenn die zweite Kontaktierungstechnik und/oder Kontaktbelegung ausgewählt wird:
- eine zweite Seite des zentralen Abschnitts, der mit den zweiten elektrischen Kontaktierungsflächen auf einer gemeinsamen Flachseite der Leiterplatte angeordnet ist, der Durchführung der bildgebenden Einheit zugewandt und mit den Kontaktöffnungen auf deren Kontaktstifte gesteckt und verlötet wird,
- der erste Arm und der zweite Arm aufeinander zu gebogen und die erste und zweite Endfläche der Arme aufeinandergelegt und miteinander verbunden werden, so dass die zweiten elektrischen Kontaktierungsflächen nach außen zeigen,
- anschließend elektrisch leitende Verbindungen der zweiten elektrischen Kontaktierungsflächen mit elektrischen Leitern eines weiteren elektrischen Verbindungselements gemäß der zweiten Kontaktierungstechnik und/oder Kontaktbelegung hergestellt werden.

Der erste und der zweite Arm werden insbesondere mittelbar aufeinander gelegt.

Das Verfahren zum Herstellen einer elektrischen Verbindung gemäß Aspekten der Erfindung ist besonders effizient durchführbar, da die Wahl der Kontaktierungstechnik zu einem späten Zeitpunkt während des Herstellungsprozesses des Endoskops, beispielsweise des Videoendoskops, getroffen werden muss. Daher ist das Verfahren schnell und flexibel an äußere Rahmenbedingungen anpassbar. Da das elektrische Verbindungsstück ein Gleichteil für eine Vielzahl von verschiedenen Typen von Endoskopen oder Videoendoskopen verwendbar ist, ist das Verfahren besonders ökonomisch durchführbar.

Das Verfahren ist insbesondere dadurch fortgebildet, dass die erste und die davon verschiedene zweite Kontaktierungstechnik aus der folgenden Liste ausgewählt werden: Verlöten, Steckverbinden, Verbinden über Kantenvias, und die ersten bzw. die zweiten Kontaktierungsflächen wahlweise mit einer dieser Kontaktierungstechniken kontaktiert werden.

Ferner ist das Verfahren dadurch fortgebildet, dass der erste Arm und der zweite Arm zumindest näherungsweise eine gleiche Länge aufweisen und zumindest abschnittsweise flächig aufeinander gelegt werden, wobei die erste und die zweite Endfläche oder die dritte und die vierte Endfläche im aufeinandergelegten Zustand von dem zentralen Abschnitt weg weisen und im aufeinandergelegten Zustand zusammen mit dem zentralen Abschnitt ein im Wesentlichen symmetrisches Dreieck bilden, wobei insbesondere die aufeinander gelegten Endflächen im Wesentlichen auf einer zentralen Achse oder Drehachse der bildgebenden Einheit angeordnet werden.

Gemäß einer weiteren vorteilhaften Ausführungsform ist vorgesehen, dass ein, insbesondere flächiger, Stabilisierungskörper zwischen den aufeinandergelegten Endflächen angeordnet wird, wobei der Stabilisierungskörper insbesondere ein elektrisch isolierendes Material umfasst und ferner insbesondere ein Material umfasst, mittels dessen ein Übersprechen von Signalen in den Leiterbahnabschnitten bzw. weiteren Leiterbahnabschnitten in den Endflächen des ersten Arms und des zweiten Arms verringert wird.

Schließlich ist das Verfahren dadurch fortgebildet, dass der erste Arm und der zweite Arm mit einem Verbindungselement verbunden werden, das die Endflächen durchdringt, wobei das Verbindungselement insbesondere beidseitig pilzköpfige Vorsprünge am Stabilisierungskörper ausgebildet.

Auch auf das Verfahren treffen gleiche oder ähnliche Vorteile zu, wie sie bereits im Hinblick auf das elektrische Verbindungsstück gemäß Aspekten der Erfindung erwähnt wurden, so dass auf Wiederholungen verzichtet werden soll.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine Seitenansicht auf ein elektrisches Verbindungsstück, welches auf eine Durchführung einer bildgebenden Einheit, beispielsweise einer Videoeinheit oder einer R-Unit aufgesetzt ist,
- Fig. 2: eine schematisch vereinfachte perspektivische Darstellung des Verbindungsstücks gemäß Fig. 1,
- Fig. 3: eine schematisch vereinfachte Darstellung des elektrischen Verbindungsstücks, welche illustriert, wie die beiden Arme in unterschiedliche Richtungen gebogen sind oder werden und
- Fig. 4: eine schematisch vereinfachte Draufsicht auf ein elektrisches Verbindungsstück, dessen Arme nicht gebogen, sondern flach ausgelegt sind.

In den Zeichnungen sind jeweils gleiche oder gleichartige Elemente und/oder Teile mit denselben Bezugsziffern versehen, so dass von einer erneuten Vorstellung jeweils abgesehen wird.

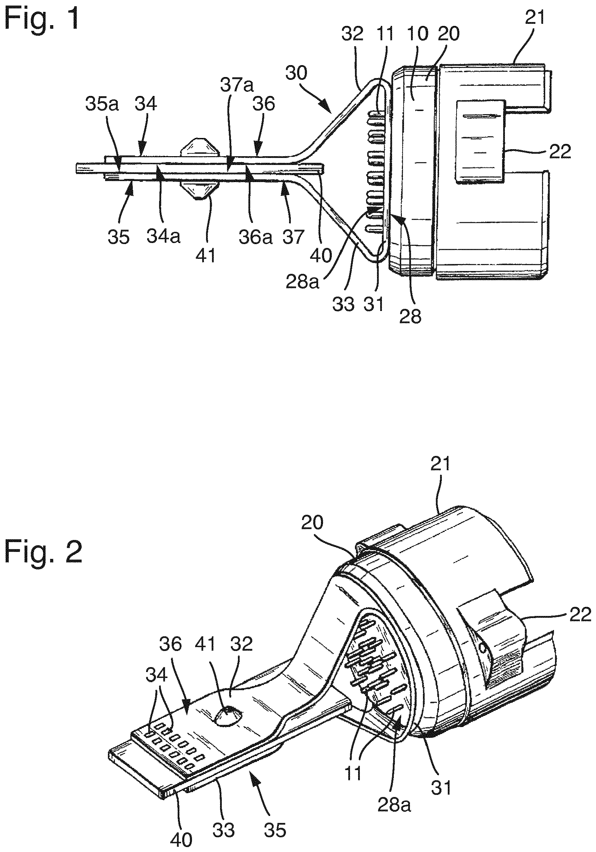

Fig. 1 zeigt ein elektrisches Verbindungsstück für ein Endoskop, beispielsweise ein Videoendoskop, schematisch und vereinfacht von der Seite dargestellt. Auf der rechten Bildseite ist eine hermetische Durchführung 10 einer bildgebenden Einheit, beispielsweise einer Videoeinheit oder einer R-Unit dargestellt. Diese ist in einem Gehäuse 20 angeordnet, an das sich proximal, in Fig. 1 rechts, ein Gehäuseteil 21 zur Aufnahme einer Steckverbindung sowie eine Schraubverbindungsstelle 22 anschließen.

Distal der hermetischen Durchführung 10, in Fig. 1 links, ragen Kontaktstifte 11 aus der Durchführung 10 heraus. Auch an der proximalen Seite stehen die Kontaktstifte 11 aus der hermetischen Durchführung 10 heraus, sie sind jedoch durch das Gehäuse 20 perspektivisch verdeckt. Die Kontaktstifte 11 sind beispielsweise in einer Glasplatte vergossen, so dass die Durchführung 10 an dieser Stelle hermetisch dicht ist.

Auf die distal herausstehenden Enden der Kontaktstifte 11 ist ein zentraler Abschnitt 31 einer flexiblen Leiterplatte 30 aufgesteckt. In dem zentralen Abschnitt 31 befinden sich in Fig. 1 nicht sichtbare Kontaktöffnungen, die in Größe und Anordnung den Kontaktstiften 11 an der hermetischen Durchführung 10 entsprechen. Bevorzugt weist der zentrale Abschnitt 31 der flexiblen Leiterplatte 30 einen Abstand zu der hermetischen Durchführung 10 auf, welcher jedoch so gering gewählt ist, dass er in Fig. 1 nicht sichtbar ist.

Die flexible Leiterplatte 30 weist einen ersten Arm 32 sowie einen zweiten Arm 33 auf, welche sich an den zentralen Abschnitt 31 anschließen. Der erste Arm 32 und der zweite Arm 33 sind biegbar ausgestaltet und erstrecken sich ausgehend von dem zentralen Abschnitt 31 in voneinander verschiedene Richtungen, bevorzugt in einander entgegengesetzte Richtungen. Der erste Arm 32 weist an seinem dem zentralen Abschnitt 31 abgewandten Ende eine erste flache Endfläche 36 auf, welche bevorzugt versteift, zumindest jedoch weniger flexibel als der übrige Arm 32, ausgestaltet ist. Ebenso weist der zweite Arm 33 an seinem dem zentralen Abschnitt 31 abgewandten Ende eine zweite flache Endfläche 37 auf, welche, ebenso wie die erste Endfläche 36, versteift, zumindest aber weniger flexibel als der zweite Arm 33, ausgeführt ist.

In der ersten und zweiten Endfläche 36, 37 sind erste elektrische Kontaktierungsflächen 34, 35 vorhanden. Zwischen den Kontaktöffnungen, die die Kontaktstifte 11 umschließen, und den ersten Kontaktierungsflächen 34, 35 verlaufen in Fig. 1 nicht dargestellte Leiterbahnen. So wird die Möglichkeit geschaffen, über die Kontaktierungsflächen 34, 35 eine elektrische Verbindung zwischen diesen und den Kontaktstiften 11 herzustellen.

Ferner ist vorgesehen, dass der erste Arm 32 eine der ersten Endfläche 36 gegenüberliegende flache dritte Endfläche 36a umfasst. Der zweite Arm 33 umfasst eine der zweiten Endfläche 37 gegenüberliegende flache vierte Endfläche 37a. In der dritten und vierten Endfläche 36a, 37a sind zweite elektrische Kontaktierungsflächen 34a, 35a vorhanden. Zwischen den zweiten elektrischen Kontaktierungsflächen 34a, 35a und den Kontaktöffnungen, in denen die Kontaktstifte 11 aufgenommen sind, verlaufen nicht dargestellte weitere Leiterbahnen.

Um die in Fig. 1 dargestellte Anordnung zu erreichen, werden der erste und der zweite Arm 32, 33 gebogen und in ihren Endbereichen mittelbar aufeinandergelegt. Zwischen den aufeinandergelegten Abschnitten der Arme 32, 33 befindet sich ein Stabilisierungskörper 40. Der Stabilisierungskörper 40 befindet sich im dargestellten Ausführungsbeispiel zwischen der dritten Endfläche 36 und der vierten Endfläche 37 und ist aus einem elektrisch isolierenden Material hergestellt. So werden die innenliegenden zweiten Kontaktierungsflächen 34a, 35a voneinander elektrisch isoliert. Bevorzugt ist der Stabilisierungskörper 40 aus einem Material hergestellt, mittels dessen ein Übersprechen von Signalen in den Leiterbahnenabschnitten bzw. weiteren Leiterbahnenabschnitten, welche sich in den Endflächen 36, 36a, 37, 37a des ersten Arms 32 und des zweiten Arms 33 erstrecken, verringert wird.

Die ersten elektrischen Kontaktierungsflächen 34, 35 sind zur Kontaktierung mit einer ersten Kontaktierungstechnik ausgestaltet. Im Gegensatz dazu sind die zweiten elektrischen Kontaktierungsflächen 34a, 35a zur Kontaktierung mit einer davon verschiedenen zweiten Kontaktierungstechnik ausgestaltet. In dem in Fig. 1 dargestellten Ausführungsbeispiel sind die Arme 32, 33 so gebogen, dass die ersten elektrischen Kontaktierungsflächen 34, 35 außen liegen, also für die Kontaktierung frei zugänglich sind.

Eine mögliche erste Kontaktierungstechnik ist beispielsweise Verlöten, Steckverbinden oder auch eine Verbindung über Kantenvias. Eine für die zweiten Kontaktierungsflächen 34a, 35a vorgesehene zweite Kontaktierungstechnik unterscheidet sich von der für die ersten Kontaktierungsflächen 34, 35 vorgesehenen ersten Kontaktierungstechnik. Sie ist bevorzugt ebenfalls aus der oben genannten Liste ausgewählt. Abhängig davon, welche Kontaktierungstechnik für das elektrische Verbindungsstück vorgesehen bzw. gewünscht ist, werden die Arme 32, 33 wahlweise in die in Fig. 1 dargestellte oder eine umgekehrte Konfiguration gebogen. Dies bedeutet, dass die dritten und vierten Endflächen 36a, 37a (wie dargestellt) aufeinander liegen und die ersten elektrischen Kontaktierungsflächen 34, 35 frei zugänglich sind oder die erste und zweite Endfläche 36, 37 aufeinander liegen und die zweiten elektrischen Kontaktierungsflächen 34a, 35a für eine Kontaktierung zugänglich sind. Entsprechend ist eine erste Seite 28 (wie dargestellt) oder eine zweite Seite 28a des zentralen Abschnitts 31 der hermetischen Durchführung 10 zugewandt.

Nach erfolgtem Biegevorgang werden die Enden der Arme 32, 33 mit einem Verbindungselement 41 miteinander verbunden. Dieses ist bevorzugt so ausgebildet, dass es zu beiden Seiten der Arme pilzköpfige Vorsprünge ausbildet. Gemäß weiterer nicht dargestellter Ausführungsbeispiele ist ebenfalls vorgesehen, die Enden der Arme 32, 33 miteinander zu verschrauben oder auch miteinander zu verkleben.

Das elektrische Verbindungsstück wird nach Kontaktierung bzw. Verbindung mit der hermetisch abgeschlossenen Durchführung 10 mit einem nicht dargestellten weiteren elektrischen Verbindungselement verbunden. Hierzu werden die ersten Kontaktierungsflächen 34, 35 bzw. die zweiten Kontaktierungsflächen 34a, 35a, in Kontakt mit dem weiteren elektrischen Verbindungselement gebracht. Beispielsweise werden sie mit einem oder mehreren Kabel(n) oder einer flexiblen Leiterplatte verlötet oder mit einem Stecker kontaktiert. In diesem Zusammenhang erleichtert die versteifte bzw. weniger flexible Ausführung der Arme 32, 33 in ihren Endbereichen die Handhabung.

Das elektrische Verbindungsstück ist bevorzugt zum Einsatz in einem Endoskop, insbesondere einem Videoendoskop, vorgesehen. Bei der bildgebenden Einheit handelt es sich beispielsweise um eine Videoeinheit oder eine R-Unit, die drehbar ausgeführt ist. Es ist in diesem Zusammenhang vorteilhaft, dass die Kontaktierungsflächen 34, 34a, 35, 35a im Wesentlichen auf einer zentralen Achse oder Drehachse der bildgebenden Einheit liegen bzw. legbar sind. Um dies zu erreichen, werden der erste Arm 32 und der zweite Arm 33 derart gebogen, dass im aufeinandergelegten Zustand der Endflächen die Arme 32, 33 gemeinsam mit dem zentralen Abschnitt 31 ein im Wesentlichen symmetrisches Dreieck bilden.

Fig. 2 zeigt die aus Fig. 1 bekannte Anordnung in einer vereinfachten schematischen und perspektivischen Ansicht. Beispielhaft sind die ersten Kontaktierungsflächen 34 des ersten Arms 32 in der ersten Endfläche 36 sichtbar. Sie sind beispielhaft als Kontakte für eine Lötkontaktierung oder für eine Kontaktierung mittels eines Steckers ausgeführt.

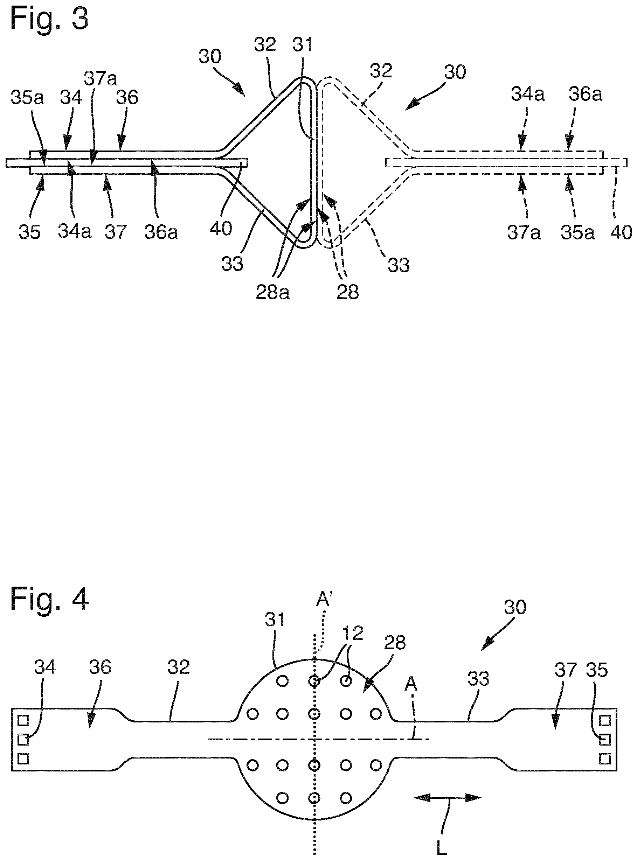

Fig. 3 zeigt in durchgezogener Linie eine schematische und vereinfachte Seitenansicht der Leiterplatte 30, deren Arme 32, 33, wie in Fig. 1 dargestellt, gebogen sind. Die ersten Kontaktierungsflächen 34, 35 befinden sich an der Außenseite der aufeinandergelegten Abschnitte der Arme 32, 33. Die erste Endfläche 36 und die zweite Endfläche 37 weisen nach außen. Das Verbindungselement 41 (vgl. Fig. 1) ist aus Gründen der Übersichtlichkeit nicht dargestellt.

Im rechten Teil von Fig. 3 ist in gestrichelter Linie die flexible Leiterplatte 30 und der Stabilisierungskörper 40 dargestellt, wobei die Arme 32, 33 in umgekehrter Richtung gebogen sind. In diesem Zustand befindet sich die dritte Endfläche 36a und die vierte Endfläche 37a an der Außenseite, so dass die zweiten Kontaktierungsflächen 34a, 35a von außen zugänglich sind.

Abhängig davon, in welche Richtung die Arme 32, 33 gebogen werden, befindet sich außerdem eine erste Seite 28 oder eine zweite Seite 28a des zentralen Abschnitts 31 an der Außenseite der Leiterplatte 30 und ist im kontaktierten Zustand der hermetisch abgeschlossenen Durchführung 10 zugewandt. In dem mit durchgezogener Linie dargestellten Zustand befindet sich die erste Seite 28 des zentralen Abschnitts 31 auf der Außenseite der Leiterplatte 30 und diese wird derart auf die Kontaktstifte 11 aufgeschoben, dass die erste Seite 28 der hermetisch abgeschlossenen Durchführung 10 zugewandt ist (vgl. auch Fig. 1). In dem in gestrichener Linie dargestellten Zustand befindet sich die zweite Seite 28a der Leiterplatte 30 an deren Außenseite. Nun sollen die zweiten Kontaktierungsflächen 34a, 35a zur Kontaktierung der Kontaktstifte 11 der hermetischen Durchführung 10 genutzt werden. Die flexible Leiterplatte 30 wird derart auf die Kontaktstifte 11 aufgeschoben, dass die zweite Seite 28a des zentralen Abschnitts 31 der hermetischen Durchführung 10 zugewandt ist.

Um wahlweise die erste oder die zweite Seite 28, 28a auf die Kontaktstifte 11 der hermetischen Durchführung 10 aufzustecken, wird die Leiterplatte 30 gewendet und ihre Arme 32, 33 werden entsprechend umgebogen. Um dennoch eine entsprechende Kontaktierung der Kontaktstifte 11 zu ermöglichen, ohne eine Fehlkontaktierung zu verursachen, ist eine Kontaktbelegung der in dem zentralen Abschnitt 31 vorgesehenen Kontaktöffnungen 12 spiegelsymmetrisch ausgeführt. Dies ist in der schematisch vereinfachten Draufsicht von Fig. 4 gezeigt. Die Fig. 4 zeigt eine Seite der Leiterplatte 30, wobei die Arme 32, 33 nicht umgebogen sind. Beispielhaft sind die Kontaktöffnungen 12 spiegelsymmetrisch zu der dargestellten Symmetrieachse A (strichpunktiert dargestellt) ausgeführt, welche näherungsweise parallel zu einer gemeinsamen Längserstreckungsrichtung L der Arme 32, 33 verläuft. Eine weitere Symmetrieachse A' (gepunktet dargestellt) verläuft, zumindest näherungsweise, senkrecht zu der Längserstreckungsrichtung L. Im dargestellten Ausführungsbeispiel sind die Anordnung der Kontakte und deren Kontaktbelegung sowohl zur Symmetrieachse A als auch zur weiteren Symmetrieachse A' spiegelsymmetrisch. So ist es möglich, die dargestellte Leiterplatte 30 wahlweise zu kippen oder zu wenden und auf die Kontaktstifte 11 wahlweise mit der ersten Seite 28 oder der gegenüberliegenden zweiten Seite 28a aufzustecken und mit diesem zu verbinden.

Gemäß einem beispielhaften Verfahren zum Herstellen einer elektrischen Verbindung in einem Endoskop, insbesondere in einem Videoendoskop, wird ein elektrisches Verbindungsstück, wie es in den Fig. 1 bis 4 gezeigt ist, bereitgestellt. Es wird ferner die Wahl zwischen der ersten und der zweiten Kontaktierungstechnik zum Kontaktieren des elektrischen Verbindungsstücks getroffen.

Wird die erste Kontaktierungstechnik gewählt, beispielsweise eine Lötverbindung, so wird die erste Seite 28 des zentralen Abschnitts 31, die mit den ersten elektrischen Kontaktierungsflächen 34, 35 auf einer gemeinsamen Flachseite der Leiterplatte 30 angeordnet ist, der Durchführung 10 der bildgebenden Einheit zugewandt und mit ihren Kontaktöffnungen 12 auf deren Kontaktstifte 11 gesteckt und verlötet. Anschließend werden der erste und der zweite Arm 32, 33 so aufeinander zugebogen, dass die dritte und die vierte Endfläche 36a, 37a der Arme 32, 33 mittelbar aufeinanderliegen. Anschließend werden die Arme 32, 33 miteinander verbunden. Zwischen den Endflächen 36a, 37a wird ein Stabilisierungskörper 40 vorgesehen. Im Ergebnis zeigen die ersten elektrischen Kontaktierungsflächen 34, 35 nach außen. Diese werden anschließend mit den elektrischen Leitern eines weiteren elektrischen Verbindungselements gemäß der ersten Kontaktierungstechnik elektrisch kontaktiert bzw. verbunden. Beispielsweise wird eine Lötverbindung hergestellt. Die ersten elektrischen Kontaktierungsflächen 34, 35 sind entsprechend für diese erste Kontaktierungstechnik, also beispielsweise eine Lötverbindung, ausgestaltet.

Wird eine zweite Kontaktierungstechnik ausgewählt, beispielsweise eine Verbindung über Kantenvias, so wird die zweite Seite 28a des zentralen Abschnitts 31, die mit den zweiten elektrischen Kontaktierungsflächen 34a, 35a auf einer gemeinsamen Flachseite der Leiterplatte 30 angeordnet ist, der Durchführung 10 der bildgebenden Einheit zugewandt und mit den Kontaktöffnungen 12 auf deren Kontaktstifte 11 gesteckt und verlötet. Anschließend werden der erste Arm 32 und der zweite Arm 33 so aufeinander zugebogen, dass die erste und die zweite Endfläche 36, 37 der Arme 32, 22, zumindest mittelbar, aufeinanderliegen. Die Arme 32, 33 werden miteinander verbunden, so dass die zweiten elektrischen Kontaktierungsflächen 34a, 35a nach außen zeigen. Anschließend wird eine elektrisch leitende Verbindung zwischen den zweiten Kontaktierungsflächen 34a, 35a, welche beispielsweise als Kantenvias ausgebildet sind, mit den elektrischen Leitern des weiteren elektrischen Verbindungselements gemäß der zweiten Kontaktierungstechnik hergestellt.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 10: hermetische Durchführung
- 11: Kontaktstifte
- 12: Kontaktöffnungen
- 20: Gehäuse
- 21: Gehäuseteil
- 22: Schraubverbindungsstelle
- 28: erste Seite
- 28a: zweite Seite
- 30: flexible Leiterplatte
- 31: zentraler Abschnitt
- 32: erster Arm
- 33: zweiter Arm
- 34, 35: erste Kontaktierungsflächen
- 34a, 35a: zweite Kontaktierungsflächen
- 36: erste Endfläche
- 36a: dritte Endfläche
- 37: zweite Endfläche
- 37a: vierte Endfläche
- 40: Stabilisierungskörper
- 41: Verbindungselement

- A, A': Symmetrieachse
- L: Längserstreckungsrichtung

## Patentansprüche

1. Elektrisches Verbindungsstück für ein Endoskop, umfassend eine wenigstens teilweise flexible Leiterplatte (30) mit einem zentralen Abschnitt (31), der von Kontaktöffnungen (12) für Kontaktstifte (11) einer hermetisch abgeschlossenen Durchführung (10) einer bildgebenden Einheit des Endoskops durchsetzt ist, wobei die Leiterplatte (30) ferner einen biegbaren ersten Arm (32) und einen biegbaren zweiten Arm (33) umfasst, die in verschiedenen Richtungen von dem zentralen Abschnitt (31) abzweigen, wobei der erste Arm (32) an seinem dem zentralen Abschnitt (31) abgewandten Ende eine erste flache Endfläche (36) umfasst und der zweite Arm (33) an seinem dem zentralen Abschnitt (31) abgewandten Ende eine zweite flache Endfläche (37) umfasst, wobei Leiterbahnen zwischen den Kontaktöffnungen (12) und in den Endflächen (36, 37) vorhandenen ersten elektrischen Kontaktierungsflächen (34, 35) verlaufen, **dadurch gekennzeichnet, dass** für die Kontaktöffnungen (12) eine bezüglich einer Symmetrieachse (A) spiegelsymmetrische Kontaktbelegung vorgesehen ist und ferner der erste Arm (32) eine der ersten Endfläche (36) gegenüberliegende flache dritte Endfläche (36a) umfasst und der zweite Arm (33) eine der zweiten Endfläche (37) gegenüberliegende flache vierte Endfläche (37a) umfasst, wobei weitere Leiterbahnen zwischen den Kontaktöffnungen (12) und den in der dritten und der vierten Endfläche (36a, 37a) vorhandenen zweiten elektrischen Kontaktierungsflächen (34a, 35a) verlaufen und wobei die ersten elektrischen Kontaktierungsflächen (34, 35) zur Kontaktierung mit einer ersten Kontaktierungstechnik und/oder Kontaktbelegung und die zweiten elektrischen Kontaktierungsflächen (34a, 35a) zur Kontaktierung mit einer davon verschiedenen zweiten Kontaktierungstechnik und/oder Kontaktbelegung ausgestaltet sind.

2. Elektrisches Verbindungsstück nach Anspruch 1, **dadurch gekennzeichnet, dass** die Arme (32, 33) in entgegengesetzten Richtungen von dem zentralen Abschnitt (31) abzweigen und die Symmetrieachse (A) zumindest näherungsweise parallel oder senkrecht zu einer gemeinsamen Längserstreckungsrichtung (L) der Arme (32, 33) verläuft.

3. Elektrisches Verbindungsstück nach Anspruch 1 oder 2, **dadurch gekennzeichnet, dass** wahlweise die ersten Kontaktierungsflächen (34, 35) in der ersten und zweiten Endfläche (36, 37) oder die zweiten Kontaktierungsflächen (34a, 35a) in der dritten und vierten Endfläche (36a, 37a) mit Kontakten eines weiteren elektrischen Verbindungselements, insbesondere eines oder mehrerer Kabel, einer oder mehrerer flexibler Leiterplatten oder eines oder mehrerer Stecker, verbindbar oder verbunden sind.

4. Elektrisches Verbindungsstück nach Anspruch 3, **dadurch gekennzeichnet, dass** die erste und die davon verschiedene zweite Kontaktierungstechnik aus der folgenden Liste ausgewählt sind: Verlöten, Steckverbinden, Verbinden über Kantenvias.

5. Elektrisches Verbindungsstück nach einem der Ansprüche 1 bis 4, **dadurch gekennzeichnet, dass** der zentrale Abschnitt Grundfläche (31) und/oder die Endflächen (36, 37) und die weiteren Endflächen (36a, 37a) der Leiterplatte (30) versteift und/oder weniger flexibel als die Arme (32, 33) ist oder sind.

6. Elektrisches Verbindungsstück nach einem der Ansprüche 1 bis 5, **dadurch gekennzeichnet, dass** der erste Arm (32) und der zweite Arm (33) zumindest näherungsweise eine gleiche Länge aufweisen und zumindest abschnittsweise flächig aufeinander legbar oder gelegt sind, wobei die erste und die zweite Endfläche (36, 37) oder die dritte und die vierte Endfläche (36a, 37a) im aufeinandergelegten Zustand von dem zentralen Abschnitt (31) weg weisen und im aufeinandergelegten Zustand zusammen mit dem zentralen Abschnitt (31) ein im Wesentlichen symmetrisches Dreieck bilden, wobei die aufeinander gelegten Endflächen (36, 36a, 37, 37a) im Wesentlichen auf einer zentralen Achse oder Drehachse der bildgebenden Einheit legbar sind oder liegen.

7. Elektrisches Verbindungsstück nach einem der Ansprüche 1 bis 6, **dadurch gekennzeichnet, dass** ein, insbesondere flächiger, Stabilisierungskörper (40) umfasst ist, der zwischen den aufeinandergelegten Endflächen (36, 36a, 37, 37a) angeordnet ist, wobei der Stabilisierungskörper (40) insbesondere ein elektrisch isolierendes Material umfasst und ferner insbesondere ein Material umfasst, mittels dessen ein Übersprechen von Signalen in den Leiterbahnabschnitten bzw. weiteren Leiterbahnabschnitten in den Endflächen (36, 36a, 37, 37a) des ersten Arms (32) und des zweiten Arms (33) verringert wird.

8. Elektrisches Verbindungsstück nach einem der Ansprüche 1 bis 7, **dadurch gekennzeichnet, dass** ein Verbindungselement (41) umfasst ist, das die Endflächen (36, 36a, 37, 37a) durchdringt und miteinander verbindet, insbesondere als beidseitige pilzköpfige Vorsprünge am Stabilisierungskörper (40) ausgebildet ist.

9. Elektrisches Verbindungsstück nach einem der Ansprüche 1 bis 8, **dadurch gekennzeichnet, dass** die Kontaktöffnungen (12) des zentralen Abschnitts (31) der Leiterplatte (30) über Kontaktstifte (11) der hermetischen Durchführung (10) steckbar oder gesteckt und mit den Kontaktstiften (11) verlötbar oder verlötet sind.

10. Endoskop, insbesondere Videoendoskop, mit einem Schaft und einer in dem Schaft angeordneten hermetisch abgeschlossenen bildgebenden Einheit, insbesondere Videoeinheit, und mit einem elektrischen Verbindungsstück nach einem der Ansprüche 1 bis 9.

11. Verfahren zum Herstellen einer elektrischen Verbindung in einem Endoskop, wobei ein elektrisches Verbindungsstück nach einem der Ansprüche 1 bis 9 bereitgestellt und die erste oder die zweite Kontaktierungstechnik und/oder Kontaktbelegung zum Kontaktieren des elektrischen Verbindungsstücks ausgewählt wird, wobei
wenn die erste Kontaktierungstechnik und/oder Kontaktbelegung ausgewählt wird:
- eine erste Seite (28) des zentralen Abschnitts (31), der mit den ersten elektrischen Kontaktierungsflächen (34, 35) auf einer gemeinsamen Flachseite der Leiterplatte (30) angeordnet ist, der Durchführung (10) der bildgebenden Einheit zugewandt und mit den Kontaktöffnungen (12) auf deren Kontaktstifte (11) gesteckt und verlötet wird,
- der erste Arm (32) und der zweite Arm (33) aufeinander zu gebogen und die dritte und vierte Endfläche (36a, 37a) der Arme (32, 33) aufeinandergelegt und miteinander verbunden werden, so dass die ersten elektrischen Kontaktierungsflächen (34, 35) nach außen zeigen,
- anschließend elektrisch leitende Verbindungen der ersten elektrischen Kontaktierungsflächen (34, 35) mit elektrischen Leitern eines weiteren elektrischen Verbindungselements gemäß der ersten Kontaktierungstechnik und/oder Kontaktbelegung hergestellt werden,
wenn die zweite Kontaktierungstechnik und/oder Kontaktbelegung ausgewählt wird:
- eine zweite Seite (28a) des zentralen Abschnitts (31), der mit den zweiten elektrischen Kontaktierungsflächen (34, 35) auf einer gemeinsamen Flachseite der Leiterplatte (30) angeordnet ist, der Durchführung (10) der bildgebenden Einheit zugewandt und mit den Kontaktöffnungen (12) auf deren Kontaktstifte (11) gesteckt und verlötet wird,
- der erste Arm (32) und der zweite Arm (33) aufeinander zu gebogen und die erste und zweite Endfläche (36, 37) der Arme (32, 33) aufeinandergelegt und miteinander verbunden werden, so dass die zweiten elektrischen Kontaktierungsflächen (34a, 35a) nach außen zeigen,
- anschließend elektrisch leitende Verbindungen der zweiten elektrischen Kontaktierungsflächen (34a, 35a) mit elektrischen Leitern eines weiteren elektrischen Verbindungselements gemäß der zweiten Kontaktierungstechnik und/oder Kontaktbelegung hergestellt werden.

12. Verfahren nach Anspruch 11, **dadurch gekennzeichnet, dass** die erste und die davon verschiedene zweite Kontaktierungstechnik aus der folgenden Liste ausgewählt werden: Verlöten, Steckverbinden, Verbinden über Kantenvias, und die ersten bzw. die zweiten Kontaktierungsflächen (34, 34a, 35, 35a) wahlweise mit einer dieser Kontaktierungstechniken kontaktiert werden.

13. Verfahren nach Anspruch 11 oder 12, **dadurch gekennzeichnet, dass** der erste Arm (32) und der zweite Arm (33) zumindest näherungsweise eine gleiche Länge aufweisen und zumindest abschnittsweise flächig aufeinander gelegt werden, wobei die erste und die zweite Endfläche (36, 37) oder die dritte und die vierte Endfläche (36a, 37a) im aufeinandergelegten Zustand von dem zentralen Abschnitt (31) weg weisen und im aufeinandergelegten Zustand zusammen mit dem zentralen Abschnitt (31) ein im Wesentlichen symmetrisches Dreieck bilden, wobei insbesondere die aufeinander gelegten Endflächen (36, 36a, 37, 37a) im Wesentlichen auf einer zentralen Achse oder Drehachse der bildgebenden Einheit angeordnet werden.

14. Verfahren nach einem der Ansprüche 11 bis 13, **dadurch gekennzeichnet, dass** ein, insbesondere flächiger, Stabilisierungskörper (40) zwischen den aufeinandergelegten Endflächen (36, 36a, 37, 37a) angeordnet wird, wobei der Stabilisierungskörper (40) insbesondere ein elektrisch isolierendes Material umfasst und ferner insbesondere ein Material umfasst, mittels dessen ein Übersprechen von Signalen in den Leiterbahnabschnitten bzw. weiteren Leiterbahnabschnitten in den Endflächen (36, 36a, 37, 37a) des ersten Arms (32) und des zweiten Arms (33) verringert wird.

15. Verfahren nach einem der Ansprüche 11 bis 14, **dadurch gekennzeichnet, dass** der erste Arm (32) und der zweite Arm (33) mit einem Verbindungselement (41) verbunden werden, das die Endflächen (36, 36a, 37, 37a) durchdringt, wobei das Verbindungselement (41) insbesondere beidseitig pilzköpfige Vorsprünge am Stabilisierungskörper (40) ausgebildet.

## Claims

1. An electrical connection piece for an endoscope comprising an at least partially flexible circuit board (30) having a central section (31) which is penetrated by contact openings (12) for contact pins (11) of a hermetically sealed feedthrough (10) of an imaging unit of the endoscope, wherein the circuit board (30) further comprises a bendable first arm (32) and a bendable second arm (33) which branch off in different directions from the central section (31), wherein the first arm (32) at its end facing away from the central section (31) comprises a first flat end surface (36), and the second arm (33) at its end facing away from the central section (31) comprises a second flat end surface (37), wherein strip conductors extend between the contact openings (12) and first electrical contacting surfaces (34, 35) present in the end surfaces (36, 37), **characterized in that** a contact assignment which is mirror-symmetrical relative to an axis of symmetry (A) is provided for the contact openings (12), and further the first arm (32) comprises a flat third end surface (36a) opposing the first end surface (36), and the second arm (33) comprises a flat fourth end surface (37a) opposing the second end surface (37), wherein further strip conductors extend between the contact openings (12) and the second electrical contacting surfaces (34a, 35a) present in the third and fourth end surfaces (36a, 37a), and wherein the first electrical contacting surfaces (34, 35) are configured for contacting using a first contact technology and/or contact assignment and the second electrical contacting surfaces (34a, 35a) are configured for contacting using a different second contact technology and/or contact assignment.

2. The electrical connection piece according to claim 1, **characterized in that** the arms (32, 33) branch off in opposing directions from the central section (31), and the axis of symmetry (A) extends at least approximately parallel or perpendicular to a common direction of longitudinal extent (L) of the arms (32, 33).

3. The electrical connection piece according to claim 1 or 2, **characterized in that** optionally the first contacting surfaces (34, 35) in the first and second end surfaces (36, 37) or the second contacting surfaces (34a, 35a) in the third and fourth end surfaces (36a, 37a) are able to be connected or are connected to contacts of a further electrical connecting element, in particular one or more cables, one or more flexible circuit boards or one or more plug connectors.

4. The electrical connection piece according to claim 3, **characterized in that** the first and the second contact technology which is different therefrom are selected from the following list: soldering, plug connection, connection by corner vias.

5. The electrical connection piece according to one of claims 1 to 4, **characterized in that** the central section, the bottom surface (31) and/or the end surfaces (36, 37) and the further end surfaces (36a, 37a) of the circuit board (30) is or are stiffened and/or less flexible than the arms (32, 33).

6. The electrical connection piece according to one of claims 1 to 5, **characterized in that** the first arm (32) and the second arm (33) have at least approximately the same length and are able to be placed or are placed at least partially flat on top of one another, wherein in the superimposed state the first and the second end surfaces (36, 37) or the third and the fourth end surfaces (36a, 37a) face away from the central section (31) and in the superimposed state together with the central section (31) form a substantially symmetrical triangle, wherein the superimposed end surfaces (36, 36a, 37, 37a) are able to be placed or are placed substantially on a central axis or rotational axis of the imaging unit.

7. The electrical connection piece according to one of claims 1 to 6, **characterized in that**, in particular, a flat stabilizing body (40) is comprised, said stabilizing body being arranged between the superimposed end surfaces (36, 36a, 37, 37a), wherein the stabilizing body (40), in particular, comprises an electrically insulating material and further, in particular, comprises a material by means of which signal crosstalk is reduced in the strip conductor portions and/or further strip conductor portions in the end surfaces (36, 36a, 37, 37a) of the first arm (32) and the second arm (33).

8. The electrical connection piece according to one of claims 1 to 7, **characterized in that** a connecting element (41) is comprised, said connecting element penetrating and connecting together the end surfaces (36, 36a, 37, 37a), in particular being configured as mushroom-shaped projections on both sides of the stabilizing body (40).

9. The electrical connection piece according to one of claims 1 to 8, **characterized in that** the contact openings (12) of the central section (31) of the circuit board (30) are able to be plugged or are plugged via contact pins (11) of the hermetically sealed feedthrough (10) and are able to be soldered or are soldered to the contact pins (11).

10. An endoscope, in particular a video endoscope, comprising a shaft and a hermetically sealed imaging unit, in particular a video unit, arranged in the shaft and comprising an electrical connection piece according to one of claims 1 to 9.

11. A method for establishing an electrical connection in an endoscope, wherein an electrical connection piece is provided according to one of claims 1 to 9 and the first or the second contact technology and/or contact assignment for contacting the electrical connection piece is selected, wherein
when the first contact technology and/or contact assignment is selected:
- a first side (28) of the central section (31), which is arranged with the first electrical contacting surfaces (34, 35) on a common flat side of the circuit board (30), faces the feedthrough (10) of the imaging unit and is placed with the contact openings (12) onto the contact pins (11) thereof and soldered,
- the first arm (32) and the second arm (33) are bent toward one another and the third and fourth end surfaces (36a, 37a) of the arms (32, 33) are superimposed and connected together so that the first electrical contacting surfaces (34, 35) face outwardly,
- subsequently, electrically conductive connections of the first electrical contacting surfaces (34, 35) with electrical conductors of a further electrical connecting element are established according to the first contact technology and/or contact assignment,
when the second contact technology and/or contact assignment is selected:
- a second side (28a) of the central section (31), which is arranged with the second electrical contacting surfaces (34, 35) on a common flat side of the circuit board (30), faces the feedthrough (10) of the imaging unit and is placed with the contact openings (12) on the contact pins (11) thereof and soldered,
- the first arm (32) and the second arm (33) are bent toward one another, and the first and second end surfaces (36, 37) of the arms (32, 33) are superimposed and connected together so that the second electrical contacting surfaces (34a, 35a) face outwardly,
- subsequently, electrically conductive connections of the second electrical contacting surfaces (34a, 35a) with electrical conductors of a further electrical connecting element are established according to the second contact technology and/or contact assignment.

12. The method according to claim 11, **characterized in that** the first contact technology and the second contact technology which is different therefrom are selected from the following list: soldering, plug connection, connection by corner vias and the first and/or the second contacting surfaces (34, 34a, 35, 35a) are optionally brought into contact by one of these contact technologies.

13. The method according to claim 11 or 12, **characterized in that** the first arm (32) and the second arm (33) have at least approximately a same length and are at least partially placed flat on top of one another, wherein the first and the second end surfaces (36, 37) or the third and the fourth end surfaces (36a, 37a) in the superimposed state face away from the central section (31) and in the superimposed state together with the central section (31) form a substantially symmetrical triangle, wherein in particular the superimposed end surfaces (36, 36a, 37, 37a) are substantially arranged on a central axis or rotational axis of the imaging unit.

14. The method according to one of claims 11 to 13, **characterized in that**, in particular, a flat stabilizing body (40) is arranged between the superimposed end surfaces (36, 36a, 37, 37a), wherein the stabilizing body (40), in particular, comprises an electrically insulating material and further, in particular, comprises a material by means of which signal crosstalk is reduced in the strip conductor portions and/or further strip conductor portions in the end surfaces (36, 36a, 37, 37a) of the first arm (32) and of the second arm (33).

15. The method according to one of claims 11 to 14, **characterized in that** the first arm (32) and the second arm (33) are connected to a connecting element (41) which penetrates the end surfaces (36, 36a, 37, 37a), wherein the connecting element (41) in particular forms mushroom-shaped projections on both sides of the stabilizing body (40).

## Revendications

1. Pièce de connexion électrique pour un endoscope, comportant une carte à circuit imprimé au moins partiellement souple (30) ayant une partie centrale (31) qui est traversée par des ouvertures de contact (12) pour des broches de contact (11) d'une traversée hermétiquement fermée (10) d'une unité d'imagerie de l'endoscope, dans laquelle la carte à circuit imprimé (30) comporte en outre un premier bras pliable (32) et un second bras pliable (33) qui bifurquent à partir de la partie centrale (31) dans des directions différentes, dans laquelle le premier bras (32) comporte, au niveau de son extrémité s'écartant de la partie centrale (31), une première surface d'extrémité plane (36) et le second bras (33) comporte, au niveau de son extrémité s'écartant de la partie centrale (31), une deuxième surface d'extrémité plane (37), dans laquelle des pistes conductrices s'étendent entre les ouvertures de contact (12) et des premières surfaces de contact électrique (34, 35) présentes dans les surfaces d'extrémité (36, 37), **caractérisée en ce que** un agencement de contacts symétrique par rapport à un axe de symétrie (A) est prévu pour les ouvertures de contact (12) et, de plus, le premier bras (32) comporte une troisième surface d'extrémité plane (36a) opposée à la première surface d'extrémité (36) et le second bras (33) comporte une quatrième surface d'extrémité plane (37a) opposée à la deuxième surface d'extrémité (37), dans laquelle des pistes conductrices supplémentaires s'étendent entre les ouvertures de contact (12) et des secondes surfaces de contact électrique (34a, 35a) présentes dans les troisième et quatrième surfaces d'extrémité (36a, 37a), et dans laquelle les premières surfaces de contact électrique (34, 35) sont configurées pour venir en contact en utilisant une première technique de contact et/ou un premier agencement de contacts et les secondes surfaces de contact électrique (34 a, 35a) sont configurées pour venir en contact en utilisant une seconde technique de contact et/ou un second agencement de contacts différent du premier.

2. Pièce de connexion électrique selon la revendication 1, **caractérisée en ce que** les bras (32, 33) bifurquent à partir de la partie centrale (31) dans des directions opposées et l'axe de symétrie (A) s'étend au moins de manière sensiblement parallèle ou perpendiculaire à une direction d'extension longitudinale commune (L) des bras (32, 33).

3. Pièce de connexion électrique selon la revendication 1 ou 2, **caractérisée en ce que** soit les premières surfaces de contact (34, 35) dans les première et deuxième surfaces d'extrémité (36, 37), soit les secondes surfaces de contact (34a, 35a) dans les troisième et quatrième surfaces d'extrémité (36a, 37a) peuvent être reliées ou sont reliées à des contacts d'un autre élément de connexion électrique, en particulier à un ou plusieurs câbles, à une ou plusieurs cartes à circuit imprimé souples ou à une ou plusieurs fiches.

4. Pièce de connexion électrique selon la revendication 3, **caractérisée en ce que** la première technique de contact et la seconde technique de contact différente de la première sont choisies dans la liste suivante : brasage, connexion par enfichage et connexion par des traversées de rebord.

5. Pièce de connexion électrique selon l'une des revendications 1 à 4, **caractérisée en ce que** la partie centrale rigidifie une surface de base (31) et/ou les surfaces d'extrémité (36, 37) et les surfaces d'extrémité supplémentaires (36a, 37a) de la carte à circuit imprimé (30) et/ou est ou sont moins souples que les bras (32, 33).

6. Pièce de connexion électrique selon l'une des revendications 1 à 5, **caractérisée en ce que** le premier bras (32) et le second bras (33) ont une longueur au moins sensiblement identique et peuvent être placés ou sont placés au moins en partie à plat l'un au-dessus de l'autre, dans laquelle les première et deuxième surfaces d'extrémité (36, 37) ou les troisième et quatrième surfaces d'extrémité (36a, 37a) s'éloignent de la partie centrale (31) à l'état superposé et forment conjointement avec la partie centrale (31) un triangle sensiblement symétrique à l'état superposé, dans laquelle les surfaces d'extrémité (36, 36a, 37, 37a) placées les unes au-dessus des autres peuvent être placées ou sont placées sensiblement sur un axe central ou un axe de rotation de l'unité d'imagerie.

7. Pièce de connexion électrique selon l'une des revendications 1 à 6, **caractérisée en ce qu'**elle comporte un corps de stabilisation (40), en particulier plat, qui est agencé entre les surfaces d'extrémité (36, 36a, 37, 37a) placées les unes au-dessus des autres, dans laquelle le corps de stabilisation (40) comporte en particulier un matériau électriquement isolant et comporte en outre en particulier un matériau au moyen duquel une diaphonie de signaux dans les parties de pistes conductrices ou des parties de pistes conductrices supplémentaires est réduite dans les surfaces d'extrémité (36, 36a, 37, 37a) du premier bras (32) et du second bras (33).

8. Pièce de connexion électrique selon l'une des revendications 1 à 7, **caractérisée en ce qu'**elle comporte un élément de connexion (41) qui pénètre dans les surfaces d'extrémité (36, 36a, 37, 37a) et les relie les unes aux autres, et est en particulier configuré comme des saillies en forme de champignon des deux côtés du corps de stabilisation (40).

9. Pièce de connexion électrique selon l'une des revendications 1 à 8, **caractérisée en ce que** les ouvertures de contact (12) de la partie centrale (31) de la carte à circuit imprimé (30) peuvent être enfichées ou sont enfichées via des broches de contact (11) de la traversée hermétique (10) et peuvent être brasées ou sont brasées avec les broches de contact (11).

10. Endoscope, en particulier un endoscope vidéo, ayant une tige et une unité d'imagerie, en particulier une unité vidéo, fermée hermétiquement et agencée dans la tige et ayant une pièce de connexion électrique selon l'une des revendications 1 à 9.

11. Procédé pour réaliser une connexion électrique dans un endoscope, dans lequel une pièce de connexion électrique est fournie selon l'une des revendications 1 à 9 et la première ou seconde technique de contact et/ou le premier ou second agencement de contacts est choisie pour mettre en contact la pièce de connexion électrique, comportant les étapes consistant à :
lorsque la première technique de contact et/ou le premier agencement de contacts est choisi :
- diriger un premier côté (28) de la partie centrale (31) qui est agencée avec les premières surfaces de contact électrique (34, 35) sur un côté plat commun de la carte à circuit imprimé (30), vers la traversée (10) de l'unité d'imagerie et enficher et braser leurs broches de contact (11) dans les ouvertures de contact (12),
- plier le premier bras (32) et le second bras (33) l'un au-dessus de l'autre et superposer les troisième et quatrième surfaces d'extrémité (36a, 37a) des bras (32, 33) et les relier l'une à l'autre de manière à diriger les premières surfaces de contact électrique (34, 35) vers l'extérieur,
- réaliser ensuite des connexions électriquement conductrices entre les premières surfaces de contact électrique (34, 35) et des conducteurs électriques d'un élément de connexion électrique supplémentaire en utilisant la première technique de contact et/ou le premier agencement de contacts,
lorsque la seconde technique de contact et/ou le second agencement de contacts est choisi :
- diriger un second côté (28a) de la partie centrale (31) qui est agencée avec les secondes surfaces de contact électrique (34, 35) sur un côté plat commun de la carte à circuit imprimé (30), vers la traversée (10) de l'unité d'imagerie et enficher et braser leurs broches de contact (11) dans les ouvertures de contact (12),
- plier le premier bras (32) et le second bras (33) l'un au-dessus de l'autre et superposer les première et deuxième surfaces d'extrémité (36, 37) des bras (32, 33) et les relier l'une à l'autre de manière à diriger les secondes surfaces de contact électrique (34a, 35a) vers l'extérieur,
- réaliser ensuite des connexions électriquement conductrices entre les secondes surfaces de contact électrique (34a, 35a) et des conducteurs électriques d'un élément de connexion électrique supplémentaire en utilisant la seconde technique de contact et/ou le second agencement de contacts.

12. Procédé selon la revendication 11, **caractérisé en ce que** la première technique de contact et la seconde technique de contact différente de la première sont choisies dans la liste suivante : brasage, connexion par enfichage ou connexion par des traversées de rebord, et les premières ou les secondes surfaces de contact (34, 34a, 35, 35a) sont sélectivement mises en contact en utilisant l'une de ces techniques de contact.

13. Procédé selon la revendication 11 ou 12, **caractérisé en ce que** le premier bras (32) et le second bras (33) ont une longueur au moins sensiblement identique et sont placées au moins en partie à plat l'un au-dessus de l'autre, dans lequel les première et deuxième surfaces d'extrémité (36, 37) ou les troisième et quatrième surfaces d'extrémité (36a, 37a) s'éloignent de la partie centrale (31) à l'état superposé et forment conjointement avec la partie centrale (31) un triangle sensiblement symétrique à l'état superposé, dans lequel les surfaces d'extrémité (36, 36a, 37, 37a) placées les unes au-dessus des autres sont en particulier agencées sensiblement sur un axe central ou un axe de rotation de l'unité d'imagerie.

14. Procédé selon l'une des revendications 11 à 13, **caractérisé en ce qu'**un corps de stabilisation (40), en particulier plat, est agencé entre les surfaces d'extrémité (36, 36a, 37, 37a) placées les unes au-dessus des autres, dans lequel le corps de stabilisation (40) comporte en particulier un matériau électriquement isolant et comporte en outre en particulier un matériau au moyen duquel une diaphonie de signaux dans les parties de pistes conductrices ou des parties de pistes conductrices supplémentaires est réduite dans les surfaces d'extrémité (36, 36a, 37, 37a) du premier bras (32) et du second bras (33).

15. Procédé selon l'une des revendications 11 à 14, **caractérisé en ce que** le premier bras (32) et le second bras (33) sont reliés à un élément de connexion (41) qui pénètre dans les surfaces d'extrémité (36, 36a, 37, 37a), dans lequel l'élément de connexion (41) est en particulier configuré comme des saillies en forme de champignon des deux côtés du corps de stabilisation (40).
